# EUROPEAN PATENT APPLICATION

(11) **EP 4 488 041 A1**
(43) Date of publication of application: **08.01.2025**
(21) Application number: 23759715.8
(22) Date of filing: 10.02.2023
(51) Int. Cl.: B29C 65/16, F16L 47/30

(54) **TUBULAR OBJECT AND PRODUCTION METHOD THEREFOR**

(30) Priority: 28.02.2022 JP 2022029374
(71) Applicant: CANON KABUSHIKI KAISHA, Tokyo 146-8501 (JP)
(72) Inventor: YAMASAKI, Toshiteru, Tokyo 146-8501 (JP); FUJINAKA, Koji, Tokyo 146-8501 (JP); SUZUKI, Akira, Tokyo 146-8501 (JP); HOSHI, Nobuharu, Tokyo 146-8501 (JP); MATSUMURA, Junichi, Tokyo 146-8501 (JP)
(74) Representative: Canon Europe Limited
(86) International application number: PCT/JP2023/004493
(87) International publication number: WO 2023/162711

(57) **Abstract**

In a catheter in which a cylindrical member 11 is assembled to an outer periphery of a tube 10 by laser welding, an outer diameter R1 of the tube 10 at a portion P1 where the cylindrical member 11 is assembled is different from an outer diameter R2 of the tube 10 at a portion P2 where the cylindrical member 11 is not assembled (R1 > R2), and the outer diameter R2 of the tube 10 at the portion P2 where the cylindrical member 11 is not assembled is smaller than an inner diameter r ≈ R1 of the cylindrical member 11. When the catheter is manufactured, the cylindrical member 11 is temporarily assembled to the outer periphery of the tube 10, air is injected into the tube 10 to bring the tube 10 into close contact with the cylindrical member 11, and laser welding is performed.

## Description

### Technical Field

The present invention relates to a tube body such as a catheter and a manufacturing method thereof.

### Background Art

Flexible medical instruments, such as endoscopic surgical instruments and catheters, are known. According to patent literatures 1 and 2, flexible medical devices that include a guide ring for guiding a wire are discussed.

Laser welding is used to assemble a cylindrical member (e.g., a member referred to as a guide ring) to an outer periphery of a tube main body (e.g., a member referred to as an inner liner or a backbone).

### Citation List

### Patent Literature

PTL 1: US No. 9144370 B1
PTL 2: WO 2018/204202 A

### Summary of Invention

### Technical Problem

In assembling a cylindrical member to an outer periphery of a tube main body by laser welding, if there is a clearance between the tube main body and the cylindrical member, an influence of the clearance may induce a welding defect.

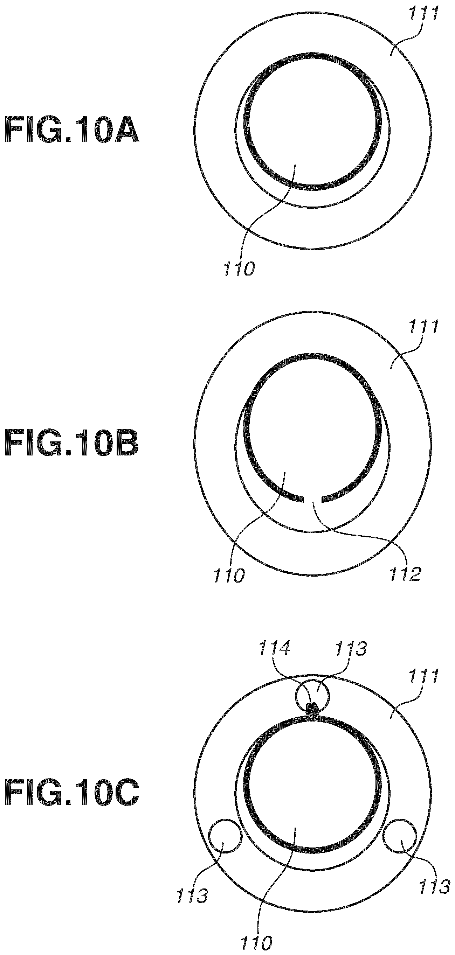

A welding defect between a tube 110 which is a tube main body and a cylindrical member 111 is described with reference to Figs. 10A, 10B, and 10C.

For example, as illustrated in Fig. 10A, for a clearance that is unevenly present, a welding defect in which the tube 110 and the cylindrical member 111 are only partially welded together.

Further, as illustrated in Fig. 10B, heat of the tube 110 that is heated by a laser is not transferred to the cylindrical member 111 due to the influence of the clearance, so that the tube 110 is overheat and a welding defect in which a hole 112 is undesirably formed. In particular, a tube for a catheter often has a thin wall thickness (e.g., about 0.25 mm), so that such a welding defect is likely to occur.

Further, as illustrated in Fig. 10C, in a configuration in which the cylindrical member 111 has wire guide holes 113 with an inner peripheral surface of the cylindrical member 111 and the wire guide holes 113 close to each other, if a laser output is increased (too much heat), the heat of the tube 110 heated by the laser may be excessively transferred to the cylindrical member 111. In this case, a wall between the wire guide hole 113 and the tube 110 melts, and melted resin 114 of the tube 110 enters the wire guide hole 113 and causes a welding defect in which the wire guide hole 113 is partially or entirely blocked.

The present invention is directed to enabling stable laser welding that is less likely to induce unnecessary welding in consideration of the above-described issues.

### Solution to Problem

A tube body according to the present invention in which a cylindrical member is assembled to an outer periphery of a tube main body by laser welding, the tube body being characterized in that an outer diameter of the tube main body in a portion where the cylindrical member is assembled is different from an outer diameter of the tube main body in a portion where the cylindrical member is not assembled, and the outer diameter of the tube main body in the portion where the cylindrical member is not assembled is smaller than an inner diameter of the cylindrical member.

### Advantageous Effects of Invention

According to the present invention, unnecessary welding is less likely to occur, and stable laser welding is enabled.

### Brief Description of Drawings

[Fig. 1A] Fig. 1A is a diagram illustrating a catheter according to a first exemplary embodiment.
[Fig. 1B] Fig. 1B is a diagram illustrating a catheter according to a first exemplary embodiment.
[Fig. 2A] Fig. 2A is a diagram illustrating a manufacturing method for the catheter according to the first exemplary embodiment.
[Fig. 2B] Fig. 2B is a diagram illustrating a manufacturing method for the catheter according to the first exemplary embodiment.
[Fig. 3] Fig. 3 is a diagram illustrating a manufacturing method for the catheter according to the first exemplary embodiment.
[Fig. 4] Fig. 4 is a diagram illustrating a catheter according to a second exemplary embodiment.
[Fig. 5] Fig. 5 is a diagram illustrating a manufacturing method for the catheter according to the second exemplary embodiment.
[Fig. 6] Fig. 6 is a characteristic diagram with time on a horizontal axis and a temperature of a tube on a vertical axis.
[Fig. 7] Fig. 7 is a diagram illustrating verification results when a rotation speed and a laser output are changed with the rotation speed × a laser irradiation time set constant.
[Fig. 8] Fig. 8 is a diagram illustrating an influence of a rotation speed.
[Fig. 9A] Fig. 9A is a diagram illustrating another configuration example of a catheter.
[Fig. 9B] Fig. 9B is a diagram illustrating another configuration example of a catheter.
[Fig. 10A] Fig. 10A is a diagram illustrating a welding defect between a tube and a cylindrical member.
[Fig. 10B] Fig. 10B is a diagram illustrating a welding defect between a tube and a cylindrical member.
[Fig. 10C] Fig. 10C is a diagram illustrating a welding defect between a tube and a cylindrical member.

### Description of Embodiments

Desirable exemplary embodiments of the present invention will be described below with reference to the accompanying drawings.

### [First Exemplary Embodiment]

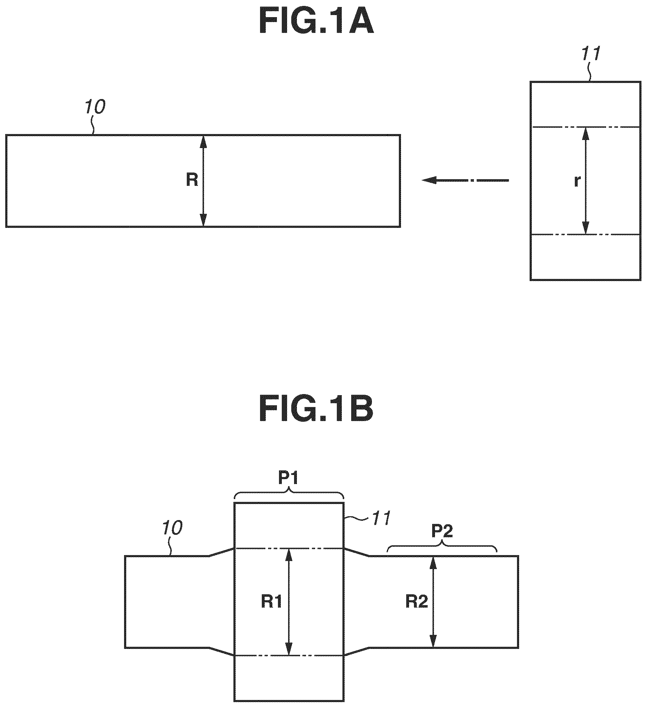

A first exemplary embodiment is described with reference to Figs. 1A to 3.

Figs. 1A and 1B are diagrams illustrating a catheter according to the first exemplary embodiment. Fig. 1A is a diagram illustrating a tube 10 and a cylindrical member 11 before assembly, and Fig. 1B is a diagram illustrating the tube 10 and the cylindrical member 11 after assembly. For the catheter, the cylindrical member 11 is assembled to an outside of the tube 10 by laser welding. According to the present exemplary embodiment, the catheter corresponds to a tube body, and the tube 10 corresponds to a tube main body according to the present invention.

Dimensions of the tube 10 alone are Φ 2.45 mm to 2.49 mm in outer diameter and Φ 2.20 mm to 2.24 mm in inner diameter. The tube 10 includes about 0.5 to 1.5% carbon black which is a laser absorber. A material of the tube 10 is nylon-based synthetic resin.

Dimensions of the cylindrical member 11a alone are Φ 3.67 mm to 3.75 mm in outer diameter and Φ 2.50 mm to 2.55 mm in inner diameter. The cylindrical member 11 is transparent so that laser can pass through it. A material of the cylindrical member 11 is nylon-based synthetic resin.

A clearance of about 0.005 mm to 0.05 mm occurs on one side in a state where the tube 10 and the cylindrical member 11 having a dimensional relationship described above are temporarily assembled.

As illustrated in Fig. 1A, for reasons that the cylindrical member 11 is assembled from an end direction of the tube 10, an outer diameter R of the tube 10 and an inner diameter r of the cylindrical member 11 have a relationship of R < r. It is desirable to bring the tube 10 and the cylindrical member 11 into close contact with each other for laser welding, but if r = R, it is difficult to assemble the cylindrical member 11 to the tube 10. Even if they can be assembled, a defect may occur in a product such as deformation in the tube 10. Thus, after the tube 10 and the cylindrical member 11 are temporarily assembled, the tube 10 and the cylindrical member 11 are to be brought into close contact with each other.

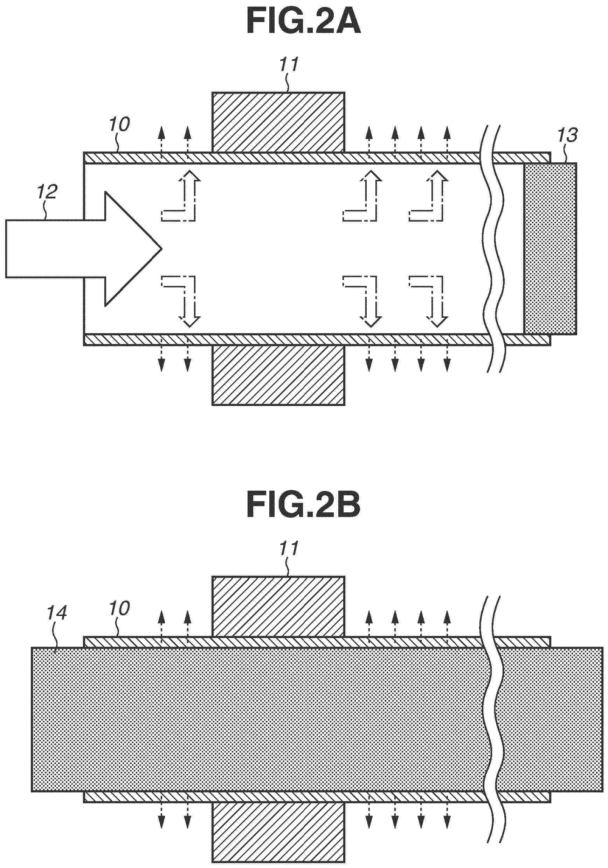

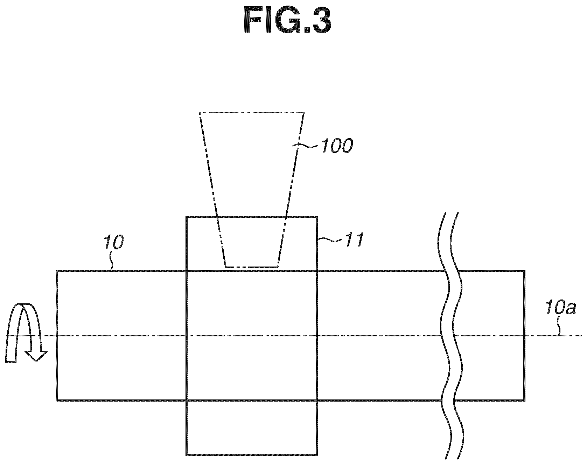

A manufacturing method for a catheter is described below with reference to Figs. 2A, 2B, and 3. Figs. 2A, 2B, and 3 are diagrams illustrating a manufacturing method for the catheter according to the first exemplary embodiment.

As illustrated in Fig. 2A, in a case where the tube 10 is made of a material that has an elastic characteristic, such as rubber, air 12 is injected into the tube 10 in a state where the tube 10 and the cylindrical member 11 are temporarily assembled. Injection of the air 12 expands the tube 10 radially outward, or a direction toward the cylindrical member 11 to bring an outer peripheral surface of the tube 10 into close contact with an inner peripheral surface of the cylindrical member 11. At this time, the air 12 is prevented from leaking out with a sealing member 13 or the like on an opposite side of a side of the tube 10 on which an inlet of the air 12 is disposed.

Further, as illustrated in Fig. 2B, a mandrel 14 having a predetermined diameter may be inserted into the tube 10 in a state where the tube 10 and the cylindrical member 11 are temporarily assembled. Insertion of the mandrel 14 expands the tube 10 radially outward, or the direction toward the cylindrical member 11 to bring the outer peripheral surface of the tube 10 into close contact with the inner peripheral surface of the cylindrical member 11.

Next, as illustrated in Fig. 3, the tube 10 and the cylindrical member 11 are laser welded while being rotated with a center 10a of the tube 10 as the center of rotation. A laser 100 passes through from a surface (outer peripheral surface) of the cylindrical member 11, melts the outer peripheral surface of the tube 10, and welds it at a contact portion with the cylindrical member 11. According to the present exemplary embodiment, laser welding is performed while the tube 10 and the cylindrical member 11 are rotated once. When the tube 10 and the cylindrical member 11 are rotated, a shaft may be inserted into the tube 10 (the mandrel 14 may also serve as the shaft), or an outer diameter portion of the tube 10 may be held. Instead of rotating the tube 10 and the cylindrical member 11, laser welding may be performed while the laser 100 is being rotated. In other words, it is sufficient that the tube 10 and the cylindrical member 11 are rotated relative to the laser 100. An entire width of the cylindrical member 11 is welded, but partial welding may be performed, or welding may be performed with a spot diameter adjusted to the width of the cylindrical member 11, if they are appropriate for weld strength and application.

Next, the air 12 and/or the mandrel 14 used for bringing the tube 10 and the cylindrical member 11 into close contact with each other is/are removed. This returns a portion having not been welded to the cylindrical member 11 to its original state, and the catheter which is a product is completed as illustrated in Fig. 1B. In the catheter, an outer diameter R1 of the tube 10 at a portion P 1 to which the cylindrical member 11 is assembled is different from an outer diameter R2 of the tube 10 at a portion P2 where the cylindrical member 11 is not assembled (R1 > R2). In addition, an outer diameter R of the tube 10 is nearly equal to R2 (R ≈ R2), and inner diameter r of the cylindrical member 11 is nearly equal to R1 (r ≈ R1). Thus, a magnitude relationship therebetween is r (R1) > R (R2).

According to the first exemplary embodiment, a rotation speed of the tube 10 and the cylindrical member 11 is set to 360 rpm as a condition for laser welding. In addition, a semiconductor laser with a wavelength of 980 nm is used for laser welding, a spot diameter is set to Φ 1.0 mm, a laser output is set to 5 W, and a laser irradiation time is set to 20 seconds. The conditions for laser welding are not limited to these, and for example, the laser irradiation time may be shortened by increasing the laser output and the rotation speed.

According to the first exemplary embodiment as described above, since laser welding is performed with the tube 10 in a close contact with the cylindrical member 11, unnecessary welding is less likely to occur, and stable laser welding is enabled.

### [Second Exemplary Embodiment]

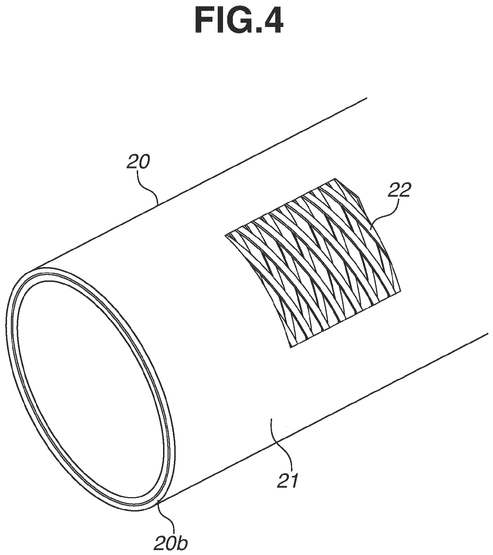

A second exemplary embodiment is described with reference to Figs. 4 to 9B. For a catheter according to the second exemplary embodiment, a cylindrical member 21 is assembled to an outside of a tube 20 by laser welding as in the first exemplary embodiment. Dimensions of the tube 20 and the cylindrical member 21 are similar to those of the tube 10 and the cylindrical member 11 according to the first exemplary embodiment.

Fig. 4 is a perspective view illustrating the catheter according to the second exemplary embodiment. According to the second exemplary embodiment, a reinforcing member 22 is incorporated in a wall 20b of the tube 20 in order to increase strength. For example, stainless steel (SUS304) having a width of 0.07 mm and a thickness of 0.03 mm is interwoven as the reinforcing member 22. Fig. 4 illustrates the wall 20b with a surface partially removed for illustration of the reinforcing member 22.

The strength of the tube 20 is thus increased so that it is difficult to deform the tube 20 by expanding it with the air 12 and/or the mandrel 14, unlike the first exemplary embodiment.

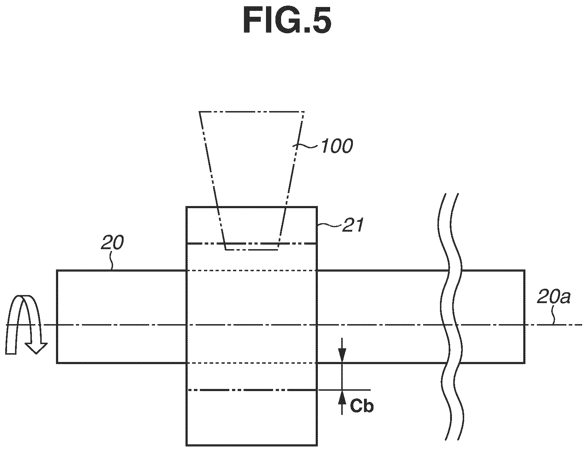

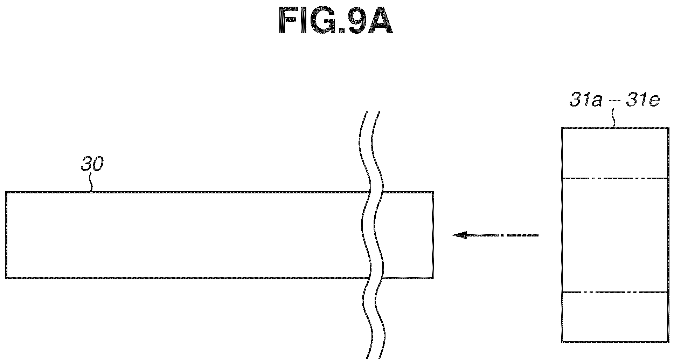

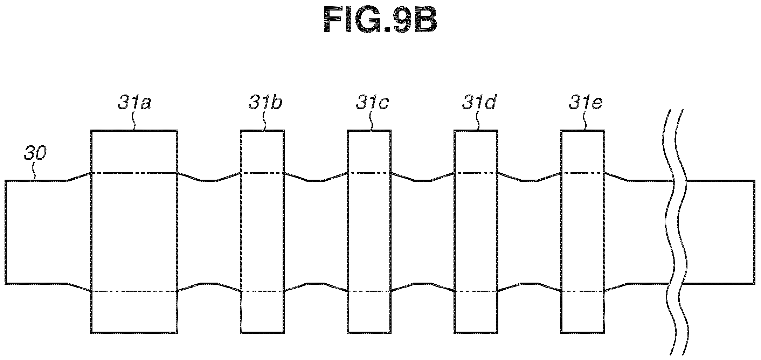

A manufacturing method for the catheter is described below with reference to Figs. 5, 9A, and 9B. Fig. 5 is a diagram illustrating the manufacturing method of the catheter according to the second exemplary embodiment.

According to the second exemplary embodiment, as illustrated in Fig. 5, the tube 20 and the cylindrical member 21 are laser welded while being rotated with a center 20a of the tube 20 as the center of rotation in a state where the tube 20 and the cylindrical member 21 are temporarily assembled. The laser 100 passes through from a surface (outer peripheral surface) of the cylindrical member 21, melts an outer peripheral surface of the tube 20, and welds it at a contact portion with the cylindrical member 21. According to the present exemplary embodiment, laser welding is performed while the tube 20 and the cylindrical member 21 are rotated a plurality of times. When the tube 20 and the cylindrical member 21 are rotated, they are fixed by a jig (not illustrated) so that a positional relationship of them is not shifted. That rotation of the tube 20 and the cylindrical member 21 relative to the laser 100 is sufficient, and a welding range are similar to those in the first exemplary embodiment.

According to the second exemplary embodiment, a rotation speed of the tube 20 and the cylindrical member 21 is set to 1800 rpm as a condition for laser welding from a viewpoint described below. In addition, a semiconductor laser with a wavelength of 980 nm is used for laser welding, a spot diameter is set to Φ 1.0 mm, a laser output is set to 16 W, and a laser irradiation time is set to 0.25 seconds.

As illustrated in Fig. 5, a clearance Cb of about 0.04 mm occurs on one side in a state where the tube 20 and the cylindrical member 21 are temporarily assembled.

According to the second exemplary embodiment, it can be said that the tube 20 and the cylindrical member 21 are welded together due to two phenomena described below.

### 1. Instantaneous heat is applied a plurality of times.

As in the first exemplary embodiment, if the outer peripheral surface of the tube 10 is in close contact with the inner peripheral surface of the cylindrical member 11, laser welding can be performed while the tube 10 and the cylindrical member 11 are rotated once.

However, if there is the clearance Cb between the tube 20 and the cylindrical member 21 as in the second exemplary embodiment, heat obtained by melting the outer peripheral surface of the tube 20 is not easily transferred to the cylindrical member 21, and welding is difficult. Thus, it is desirable that the outer peripheral surface of the tube 20 is gradually melted so as to achieve a state where the melted resin fills the clearance Cb. In order to achieve this, the tube 10 and the cylindrical member 11 are laser welded while being rotated a plurality of times so that instantaneous heat is applied a plurality of times.

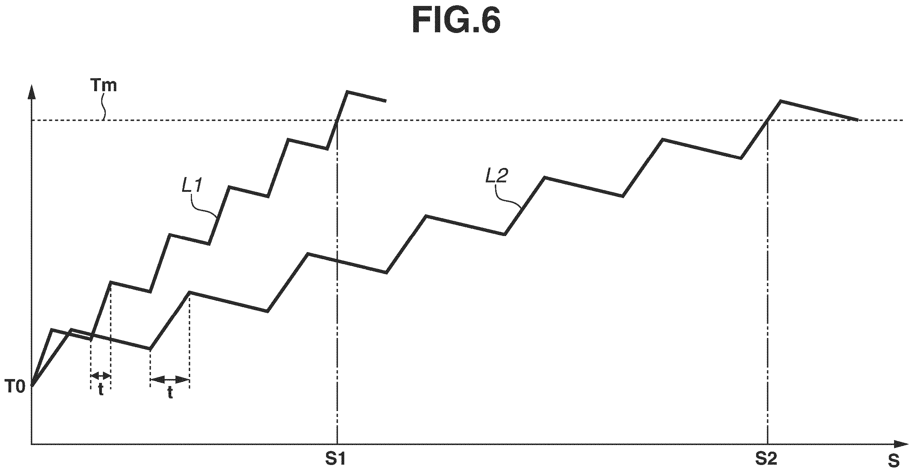

Thus, a temperature rise at a given position on the tube 20 was estimated when laser welding was performed with the rotation speed of the tube 20 and the cylindrical member 21 changed. Fig. 6 is a characteristic diagram in which the horizontal axis represents time S, and the vertical axis represents temperature T at a given position on the tube 20, and illustrates graphs L1 and L2 for different rotation speeds. Preconditions were that a temperature rise in one laser irradiation was set constant (constant heat quantity), and an inclination of cooling was also set constant, assuming heat transfer through the tube 20.

As illustrated in Fig. 6, a temperature of the tube 20 rises and falls repeatedly and reaches a temperature Tm at which the tube 20 melts. A time during which the temperature rises with one laser irradiation (t in Fig. 6) is defined as a welding time. In the graph L1 for a high rotation speed, the temperature Tm at which the tube 20 melts is reached at time S1. In contrast, in the graph L2 for a low rotation speed, the temperature Tm at which the tube 20 melts is reached at time S2 (> S1). In a case where it takes a long time to reach the temperature Tm at which the tube melts, influences of other factors (convection and heat dissipation into the air) are to be considered. Thus, according to the present exemplary embodiment, the rotation speed is set to 1800 rpm, and the laser irradiation time is set to 0.25 seconds with the graph L1 for a high rotation speed in mind.

Here, as illustrated in Fig. 6, a cooling time (a time from when a certain point is laser-irradiated to when the point is laser-irradiated next) in which the temperature falls is longer if the rotation speed is slower. Thus, as the rotation speed is slower, a required heat is higher. In other words, as the rotation speed is faster, the required heat is lower. A total heat quantity in a case where the rotation speed is 1800 rpm and the laser output is 16 W is set to "laser irradiation time 0.25 s × laser output 16 W = total heat quantity 4". If this is considered to be the center, when the rotation speed < 1800 rpm is satisfied, the cooling time increases, so that the total heat quantity > 4 is set, whereas when the rotation speed > 1800 rpm is satisfied, the cooling time decreases, so that the total heat quantity < 4 is set. In this manner, the rotation speed of the tube 20 and the cylindrical member 21 is set in a relationship in which as the rotation speed is higher, the total heat quantity is lower.

### 2. Influence of the rotation speed

An influence of the rotation speed of the tube 20 and the cylindrical member 21 was examined.

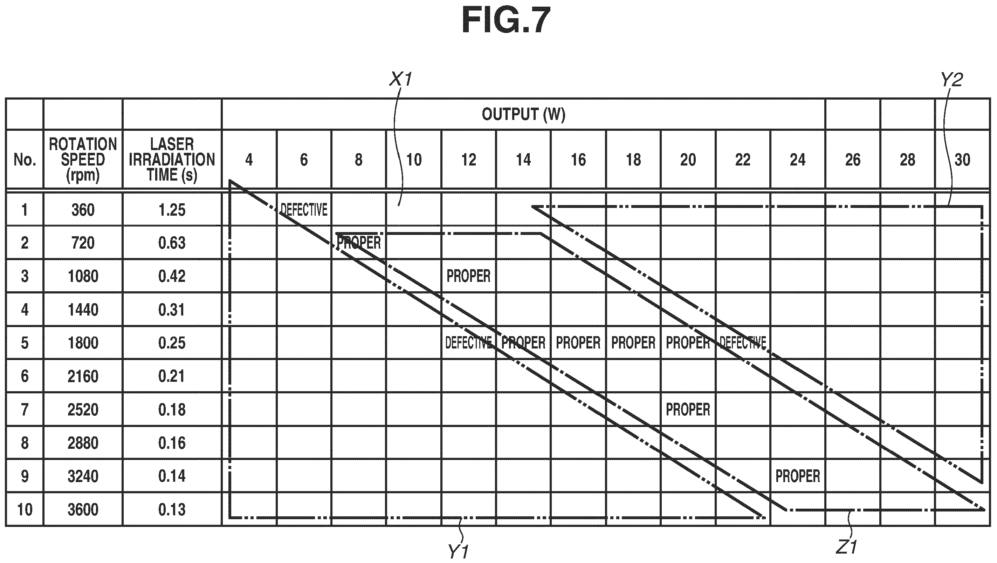

Fig. 7 is a diagram illustrating results obtained by verifying whether laser welding is proper or defective, with the rotation speed and the laser output changed and with a multiplication value of the rotation speed and the laser irradiation time (rotation speed × laser irradiation time) set constant. That the rotation speed × the laser irradiation time is set constant does not mean that the values are made completely the same, but rather that a certain degree of difference in the values is permitted, and it is sufficient that the values are approximately constant. As illustrated in Fig. 7, it was found that when the laser welding was performed while the rotation speed and the laser output were being changed with the rotation speed × the laser irradiation time set constant, a welding defect occurred unless the rotation speed was increased to a certain extent.

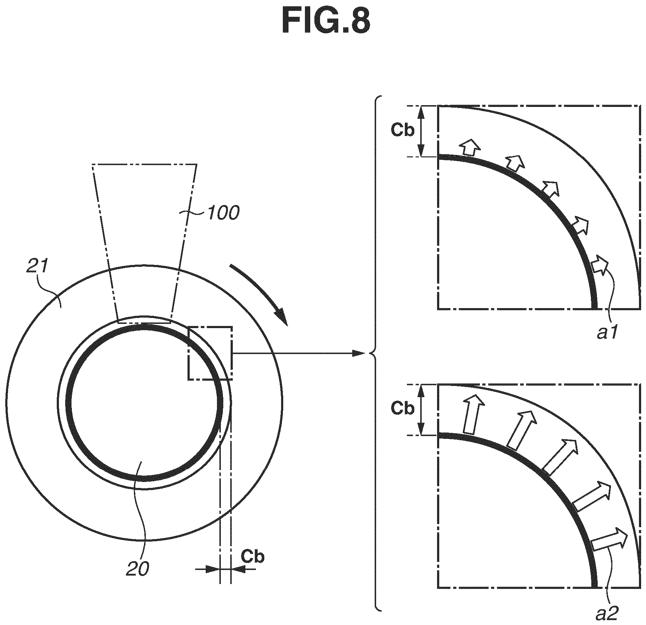

At the rotation speed of 360 rpm, even if the laser output was increased, a welding defect as illustrated in Fig. 10A occurred. Then, a condition where no welding defect occurs appeared at the rotation speed of 720 rpm or more. Fig. 8 is a diagram illustrating an influence of the rotation speed. As illustrated in Fig. 8, it is inferred that the outer peripheral surface of the tube 20 melts, the melted resin is moved outward by centrifugal force, and the heat of the melted resin melts an inner peripheral surface of the cylindrical member 21, resulting in welding. At the rotation speed of 360 rpm, as illustrated in an upper right diagram in Fig. 8, a movement amount a1 of the resin gradually melted by the laser 100 was insufficient to fill the clearance Cb, so that the entire circumference was not welded, and even if it was welded, it was only partially. In contrast, at the rotation speed of 720 rpm or more, as illustrated in a lower right diagram in Fig. 8, it can be said that a movement amount a2 of the resin gradually melted by the laser 100 is sufficient to fill the clearance Cb.

As illustrated in Fig. 7, a weldable area Z1 can be predicted by verifying whether laser welding is proper or defective with the rotation speed and the laser output being changed with the rotation speed × laser irradiation time set constant. As described above, at the rotation speed of 1800 rpm, which is set with the graph L1 for a high rotation speed in mind, the weldable area Z 1 falls a range in which the laser output is 14 W or more and 20 W or less.

Non-weldable areas are roughly classified into a non-weldable area Y1 on a lower laser output side and a non-weldable area Y2 on a high laser output side. In the non-weldable area Y1, the tube 20 and the cylindrical member 21 are not welded to each other due to insufficient heat (resin does not melt sufficiently). In contrast, in the non-weldable area Y2, an excess heat state (excessive melting of the resin) may arise to cause a defect as illustrated in Fig. 10B. Further, if there is a wire guide hole in the cylindrical member 21, a defect illustrated in Fig. 10C may occur.

As described above, the rotation speed of the tube 20 and the cylindrical member 21 is set in advance such that the conditions within the weldable area Z1 are selected based on the verification results as illustrated in Fig. 7. If the laser irradiation time or the clearance Cb is changed, the weldable area Z1 and the non-weldable areas Y1 and Y2 each presumably change (shift). In particular, if the clearance Cb is small, it can be inferred that a weldable condition appears even in conditions of a low rotation speed area X1.

According to the second exemplary embodiment as described above, even if there is a clearance between the tube 20 and the cylindrical member 21, unnecessary welding is less likely to occur and stable laser welding can be performed by the rotation speed and the laser output appropriately set.

Even in a case where a plurality of cylindrical members 31a to 31e (in various clearance relationships) is assembled outside a tube 30 by laser welding as illustrated in Figs. 9A and 9B, application of the present exemplary embodiment makes unnecessary welding less likely to occur, thus enabling stable laser welding.

According to the second exemplary embodiment, an example targeted at a catheter incorporating a reinforcing member has been described, but the manufacturing method for a catheter described according to the second exemplary embodiment is also applicable to a case where a reinforcing member is not incorporated.

The present invention is described above together with the exemplary embodiments, but the above-described exemplary embodiments are merely examples for implementing the present invention, so that the exemplary embodiments should not be construed restrictively limiting the technical scope of the present invention. In other words, the present invention can be implemented in the various forms without departing from the technical idea or the main features thereof. For example, the numerical values described in the exemplary embodiments are for reference numerical values and do not limit the present invention.

The present invention is not limited to the above-described exemplary embodiments, and various modifications and changes can be made without departing from the spirit and the scope of the present invention. Therefore, the following claims are attached in order to publicize the scope of the present invention.

This application claims priority based on Japanese Patent Application No. 2022-029374 filed on February 28, 2022, which is hereby incorporated by reference herein in its entirety.

### Description of Reference Numerals

10, 20, 30 Tube
11, 21, 31a to 31e Cylindrical member
12 Air
14 Mandrel
100 Laser

## Claims

1. A tube body in which a cylindrical member assembled to an outer periphery of a tube main body by laser welding, the tube body being **characterized in that** an outer diameter of the tube main body in a portion where the cylindrical member is assembled is different from an outer diameter of the tube main body in a portion where the cylindrical member is not assembled, and the outer diameter of the tube main body in the portion where the cylindrical member is not assembled is smaller than an inner diameter of the cylindrical member.

2. The tube body according to Claim 1, wherein a reinforcing member is incorporated in a wall of the tube main body.

3. A method for manufacturing the tube body according to Claim 1, the method being **characterized in that** the cylindrical member is temporarily assembled to the outer periphery of the tube main body, air is injected into the tube main body to bring the tube main body into close contact with the cylindrical member, and laser welding is performed.

4. A method for manufacturing the tube body according to Claim 1, the method being **characterized in that** the cylindrical member is temporarily assembled to the outer periphery of the tube main body, a mandrel is inserted into the tube main body to bring the tube main body into close contact with the cylindrical member, and laser welding is performed.

5. A method for manufacturing the tube body according to Claim 1 or 2, the method being **characterized in that** the cylindrical member is temporarily assembled to the outer periphery of the tube main body, and laser welding is performed while the tube main body and the cylindrical member, and laser are rotated relative to each other at a predetermined rotation speed for a plurality of rotations with a center of the tube main body serving as a center of rotation.

6. The method for manufacturing the tube body according to Claim 5, wherein the predetermined rotation speed is set in advance based on a result obtained by verifying whether laser welding is proper or defective with a rotation speed and laser output changed and with a multiplication value of the rotation speed and a laser irradiation time set constant.

7. The method for manufacturing the tube body according to Claim 5 or 6,
wherein a multiplication value of a laser irradiation time and laser output is regarded as a total heat quantity, and
wherein the predetermined rotation speed is set in a relationship in which as the rotation speed is faster, the total heat quantity is lower.
